# EUROPEAN PATENT APPLICATION

(11) **EP 1 479 783 A2**
(43) Date of publication of application: **24.11.2004**
(21) Application number: 04252940.4
(22) Date of filing: 18.05.2004
(51) Int. Cl.: C12Q 1/68

(54) **PCR amplification method, pcr primer set, pcr amplification product, and method for detection of nucleic acid using the amplification method**

(30) Priority: 19.05.2003 JP 2003140836; 19.05.2003 JP 2003140838; 04.03.2004 JP 2004061117
(71) Applicant: CANON KABUSHIKI KAISHA, Tokyo (JP)
(72) Inventor: Suzuki, Tomohiro, c/o Canon Kabushiki Kaisha, Tokyo (JP); Kawaguchi, Masahiro, c/o Canon Kabushiki Kaisha, Tokyo (JP); Ishii, Mie, c/o Canon Kabushiki Kaisha, Tokyo (JP)
(74) Representative: Beresford, Keith Denis Lewis

(57) **Abstract**

A nucleic acid amplification method capable of preferentially amplifying a nucleic acid strand having a desired nucleotide sequence from a template nucleic acid molecule using a PCR amplification method, where the two primers flanking a base sequence region to be amplified are each designed so that they have different template-primer melting temperature (Tm) under PCR conditions.

## Description

### BACKGROUND OF THE INVENTION

### Field of the Invention

The present invention relates to a method for preferentially amplifying a nucleic acid strand having a desired nucleotide sequence of a template nucleic acid using a PCR amplification method. The present invention also relates to a PCR primer set that is used in such a PCR amplification method, a PCR amplification product obtained by using the method, and a method for detection of nucleic acid using the amplification method.

### Related Background Art

In recent years, with rapid progress in genetic sequence analysis technology, many genetic sequences, especially of human genome, have been determined, and in vivo functions of proteins and nucleic acids coded by the deciphered genes have been elucidated. As a result, it has been elucidated that gene expression states, mutation of the base sequences or mutation of the coded amino acid sequences are related to various diseases and physical constitution. With accumulation of such genetic information, there are increasing needs for technologies for detecting a nucleic acid molecule having a specific base sequence in a sample with high accuracy. In response to the needs, various related techniques applicable to the detection technology have been developed.

Among various nucleic acid detection methods, the most frequently used method is a hybridization method. In this detection method utilizing hybridization, a nucleic acid probe having a sequence complementary to the base sequence of a nucleic acid molecule to be detected is prepared and the probe immobilized on a substrate or in a liquid is subjected to hybridization reaction with the nucleic acid in the sample, and the resulted hybrid is detected by a suitable method to determine the presence or absence of the target nucleic acid in the sample, or further to detect it quantitatively.

Detection of a nucleic acid molecule having a specific base sequence contained in a specimen is useful for diagnosis of various genetic diseases using marker genes. Further, since correlation between specific nucleic acid molecules contained in a specimen and a certain disease such as cancers and infectious diseases has been elucidated recently, its application to diagnosis of a wide range of diseases is expected.

In the nucleic acid detection methods based on hybridization, a technique utilizing a DNA chip or DNA micro-array has been widely used recently. The DNA chip or DNA micro-array method is such that a synthetic DNA chain (probe) complementary to a base sequence of a nucleic acid molecule to be detected (target nucleic acid) is previously immobilized on a solid surface such as a glass substrate, the probe is subjected to hybridization reaction on the substrate with sample nucleic acid previously labeled with a fluorescent substance or the like, and the presence or absence of the target nucleic acid in the sample or quantity thereof is determined by measuring fluorescence intensity of the hybridized probe-labeled nucleic acid. As a result of technical development in fabrication of DNA chips, many kinds of nucleic acid probes (DNA probes) can be arranged at a high density. Thus this method is highly expected as an epoch-making technique applicable to detection of a large number of items with high sensitivity at a time.

The DNA chip allows detection with high sensitivity. However, the absolute amount of the target nucleic acid in a specimen is generally very small, and the target nucleic acid should be labeled with a labeling substance such as a fluorescent material by an appropriate method. Thus, in detection of a nucleotide sequence using the DNA chip, the specimen treatment technique is very important along with the technique of fabricating the DNA chip.

For nucleic acid amplification, a method called Polymerase Chain Reaction (PCR) is most widely used. The PCR method is a method in which a specific sequence in a sample is amplified at a very high amplification rate. The PCR method is described in U.S. Patent No. 4683195, 4683202, 4965188 and so on.

When sample preparation is carried out for substrate hybridization such as the DNA chip by PCR, first a region to be amplified is determined so as to include the region corresponding to the probe immobilized on the substrate, and primers are designed at both ends of the amplification region. The PCR reaction is carried out using the both designed primers to synthesize a large amount of double-stranded nucleic acid including a desired probe-corresponding region. If it is desired to label the specimen with a certain labeling substance, a labeled monomer is added to the substrates of PCR (i.e. mononucleotides) at a predetermined ratio, or the primer is labeled with a certain labeling substance to obtain labeled double-stranded nucleic acid.

When a nucleic acid molecule having a specific base sequence is detected using the hybridization reaction, the larger the amount of single-strand nucleic acid molecules that forms a hybrid with the probe, the more accurately the nucleic acid molecule can be detected. Usually, the content of the target nucleic acid molecule in a primary sample is often insufficient. Thus, usually a sample containing a target nucleic acid quantitatively amplified according to the content of the target nucleic acid in the primary sample in advance, and then the detection of the nucleic acid is carried out. In a step of preparing the sample, it is desirable that the DNA strand being complementary to the probe is synthesized in a large amount. That is, it is desirable that, of the double strands of a sample DNA, the DNA strand containing a portion complimentary to the probe (target strand) is present in an amount larger than the opposite DNA strand containing the same sequence as the probe, for efficiency of subsequent hybridization reaction. This is not simply because a higher concentration of the target strand is desirable for detection sensitivity of hybridization, but also because the DNA strand having the same base sequence as the probe (anti-target strand) will compete with the target strand in the hybridization reaction. Thus, by relatively reducing the concentration of the anti-target DNA, efficiency of the hybridization reaction of the target strand with the probe will be increased. From these two reasons, it is desired that the content of the target strand is higher than that of the anti-target strand.

For amplifying one of the DNA strands efficiently, there are various methods. A typical method is asymmetric PCR in which the content of the primer used for extension of the target strand is made relatively high. Another method is the two-stage PCR in which double-stranded DNA containing the target strand is synthesized by the usual PCR amplification reaction, and then only the primer for target strand extension is added, and the amplification reaction is carried out again.

These conventional methods, however, often cannot achieve preferential amplification of the desired target strand owing to various factors. For example, the asymmetric PCR often suffers from the problems that the yield of the PCR amplification product is insufficient, and if the yield is sufficient, PCR amplification is not asymmetric resulting in production of mostly double-stranded DNA. The two-stage PCR method is complicated because the PCR reaction proceeds in two stages. In addition, the overall amplification yield tends to vary causing a problem in reproducibility. Further, according to this method, only one strand is added as the primer in the second-stage PCR reaction. In some cases, the second-stage PCR reaction does not proceed at all. Thus, development of a method capable of carrying out preferential amplification synthesis of a desired single-strand DNA (target strand) more conveniently and with high reproducibility is desired.

### SUMMARY OF THE INVENTION

The present invention provides a preferential nucleic acid amplification method more convenient and highly reproducible, which can preferentially amplify a nucleic acid strand having a desired nucleotide sequence from a template nucleic acid molecule using the PCR amplification method.

The inventors have intensively studied a more convenient and more reproducible method of preferentially amplifying a nucleic acid strand having a desired nucleotide sequence from a template nucleic acid by PCR, which is applicable to preparation of a sample for detection of a nucleic acid molecule having a specific base sequence, utilizing a hybridization reaction. The inventors have found out that if the base sequences of two primers for a region to be amplified are designed in such a manner that the melting temperature (Tm) of these two primers are different each other under the PCR reaction condition, the PCR amplification reaction with these primers enables amplification of respective strands of the template DNA at different yields with high reproducibility. In other words, the inventors have found out a method for preferential amplification of a single-stranded DNA starting from one primer (primer-extended strand). Here Tm value of a primer means the melting temperature of the double-stranded primer, that is, a hybrid between the primer and the corresponding complementary portion of the template. Particularly, the inventors confirmed that when the anneal temperature in the PCR amplification reaction was set to between the Tm values of two primers designed to be mutually different in Tm, the strand extended from the high Tm primer was amplified at a remarkably high efficiency compared to that from the low Tm primer in PCR. Thus the present invention was completed.

Specifically, one embodiment of the present invention is a method for producing an amplification product having a specific base sequence from a nucleic acid molecule in a sample by PCR amplification using a primer set comprised of at least two primers,
wherein at least one primer of the primer set has a melting temperature (Tm) different from the other primer or primers under conditions of the PCR amplification.

Furthermore, another embodiment of the present invention is a method for amplifying at least one specific nucleotide sequence of a nucleic acid contained in a sample by a PCR method using a primer set of at least two primers, wherein one primer of the primer set for extending a strand having a specific nucleotide sequence has a Tm value different from the other primer or primers under annealing conditions of PCR, the amplification method comprising the steps of:
carrying out the PCR having an annealing step at a temperature equal to or less than the Tm values of primers constituting the primer set in a reaction solution containing said sample and said primer set to obtain a reaction solution having amplified fragments containing said specific nucleotide sequence; and
carrying out the PCR having an annealing step at a temperature equal to or less than the Tm value of the primer for extending the fragment having the specific nucleotide sequence and higher than the Tm values of the other primers, which are among primers constituting the primer set in the reaction solution, for the reaction solution having amplified fragments having said specific nucleotide sequence, to further amplify the fragment having the specific nucleotide sequence.

According to the nucleic acid amplification method of the present invention, it is possible to preferentially synthesize one of the two strands of the template nucleic acid by appropriately determining: the Tm values of two or more primers for PCR and the annealing temperature in the amplification reaction. Furthermore, the preferential amplification of a desired strand according to the present invention is suitably applicable to preparation of a sample for nucleic acid detection assay using a DNA chip, where efficient synthesis of a DNA strand containing a sequence complementary to the probe sequence is desired.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

The accompanying drawings, which are incorporated in and constitute a part of the specification, illustrate embodiments of the invention and, together with the description, serve to explain the principles of the invention.
FIG. 1 schematically shows the basic technical principle of the nucleic acid amplification method according to the present invention;
FIG. 2 shows an electrophoretic pattern of a purified PCR amplification product obtained in Example 1; and
FIG. 3 shows the result of electrophoresis of the purified PCR amplification product obtained in Example 5.

### DESCRIPTION OF THE PREFERRED EMBODIMENTS

Preferred embodiments of the present invention will now be described in detail in accordance with the accompanying drawings.

The following, are descriptions of practical forms of the nucleic acid amplification method, the primer set for the nucleic acid amplification method, the PCR amplification product obtained by application of the nucleic acid amplification method, and the nucleic acid detection method according to the present invention.

First, according to one preferred embodiment of the present invention, a primer having a higher Tm value than the other primer(s) in the primer set is used for extending a nucleic acid strand of which preferential amplification is desired. In addition, if the sample contains two or more kinds of nucleic acid molecules, the nucleic acid amplification method preferably further comprises the steps of:
selecting one or more nucleic acid molecules to be amplified from plural nucleic acids contained in the sample;
selecting one or more base sequence regions to be amplified from entire base sequences of the selected nucleic acid molecules;
designing a forward primer and a reverse primer for PCR amplification for each of the selected base sequence regions as a primer set; and
selecting one or more strands to be extended with preferential amplification, from two or more kinds of strands to be extended from the primers of the primer set in the PCR amplification reaction.

For example, when a PCR amplification is carried out to amplify a specific base sequence of a nucleic acid in a sample using two primers, the strand containing the desired specific sequence can be preferentially amplified by using a primer having a higher Tm value than the other primer, from which the desired strand is extended.

As an embodiment, the temperature of the annealing step where the primers bind to the template in PCR is set in a temperature range between the lowest Tm and the highest Tm of the primers of the primer set used. More specifically, when two primers are used for PCR amplification of a specific base sequence of a nucleic acid in a sample, the temperature of the annealing step is set in a range between the two Tm values of the primers.

Furthermore, for effectively carrying out the nucleic acid amplification method according to the present invention, it is possible to carry out a general amplification step containing a first annealing step where annealing is carried out at an annealing temperature not higher than the both Tm values of the two primers and a target strand-synthesizing step containing a second annealing step where annealing is carried out at a temperature between the Tm values of the two primers. These two steps can be carried out separately, but they can be easily switched over through the temperature control device for the PCR reaction system.

That is, when at least one specific nucleotide sequence of a nucleic acid in a sample by the PCR method is amplified using a primer set made of at least two primers having different Tm values, the amplifying method may comprises the steps of:
carrying out a PCR in which an annealing step is carried out at a temperature not higher than either of the Tm values of the primers to obtain a reaction solution containing amplified fragments containing the specific nucleotide sequence; and
carrying out a PCR in which the annealing step is carried out at a temperature not higher than the Tm value of the primer that is used for extending the strand of the desired sequence and not lower than the Tm value of the other primer, to amplify the strand having the desired specific sequence preferentially.

In the above embodiment, the first amplification and the second amplification can be carried out in succession without any purification, and by the second amplification step the strand extended from the primer having the higher Tm value can be amplified in an amount larger than that of the extended strand from the primer having the lower Tm value (target strand synthesizing step).

Efficient amplification and labeling of the target strand are possible by the conventional two-stage PCR in separate steps using separate reaction vessels. In this embodiment, however, the similar target strand amplification can be carried out in the same vessel, that is, first, PCR reaction is carried out at an annealing temperature not higher than the Tm value of the primer for extending the complementary strand of the target strand (low Tm primer) to amplify the double-stranded nucleic acid, and then PCR reaction is carried out at an annealing temperature between the Tm value of the primer for extending the target strand (high Tm primer) and the Tm value of the low Tm primer, whereby the amount of the target strand can be increased preferentially.

By setting the Tm values of the primers for PCR amplification and the annealing temperature in the PCR appropriately, the target strand can be efficiently amplified in a large amount. In many cases, the Tm value of the primer for the target strand is designed to be higher than the annealing temperature, and the Tm value of the primer for the complementary strand of which amplification is not desired is set lower than the annealing temperature, whereby only the desired DNA can be efficiently amplified. This can also be used in the multiplex PCR using a plurality of primer sets at a time.

The nucleic acid amplification method according to the present invention may be used in sample preparation involving amplification of the sample nucleic acid to be hybridized to a DNA chip fabricated for detection of the nucleic acid.

That is, the present invention enables efficient amplification of the DNA strand that specifically hybridizes to the probe DNA on the DNA chip, and labeling of the amplified product by incorporation of a labeling substance during the amplification process, using labeled primers or using a labeled nucleotide substrate.

Thus, as described above, according to the present invention, by setting the primer Tm values and the annealing temperature in PCR appropriately, a desired primer-extended strand (target strand) can be efficiently synthesized in preference to the complementary strand by PCR, even if the amount of the nucleic acid having a target sequence in a sample is small.

The nucleic acid amplification of the present invention is possible as long as the Tm values of at least two primers are appropriately different under PCR reaction conditions.

More specifically, a temperature difference between the Tm values exhibited by the two primers is more preferably in the range of 10 to 20°C, but it is also preferable the temperature difference between the Tm values exhibited by the two primers is in the range of 5 to 20°C. It is also possible that the temperature difference in the Tm values exhibited by the two primers is in the range of 1 to 5°C.

A primer set having at least one primer different in Tm value under PCR amplification conditions from other primers can be obtained, for example, by selecting the base sequence of the primer to have different GC content than the other primer(s) to affect the Tm value. Alternatively, the length of the primers are adjusted so that at least one primer of the primer set may have a Tm value different from other primers.

Alternatively, by subjecting the primer to a certain chemical modification, the Tm value of the primer can be adjusted. This is based on the phenomenon that the chemical modification group affects stability of the double-stranded structure of the primer and the template strand thereof and as a result, the Tm value of the primer is changed. Specifically, if the introduced chemical modification group enhances the stability of the double strand, the Tm value will increase, and conversely if it reduces the stability, the Tm value will decrease.

Adjustment of the Tm value by the chemical modification group can considerably affect the double-strand stability especially when the chemical modification has a property as an intercalater or a groove binder. Thus, by introducing a chemical modification group functioning as an intercalater or groove binder, the Tm value of the primer can be adjusted.

The present amplification method may have a step of comparing the Tm values of at least two primers of the primer set under conditions of the PCR amplification reaction.

To compare Tm values of the primers, Tm values can be calculated based on the base sequences using the nearest neighbor method, the Wallace method, or the CG% method.

In the nucleic acid amplification method according to the present invention, the amplification product obtained by PCR amplification may include an amplification product labeled with a labeling substance.

For example, a labeled deoxynucleotide may be incorporated in the substrate deoxynucleotides for PCR amplification so that the amplification product has a label derived from the labeled deoxynucleotide in the extended strand. Alternatively, the amplification product may be labeled with a previously labeled primer added to the primer set, and the amplification product is extended from the labeled primer by PCR, as in the case of the PCR using the chemically modified primer. In that case, 5' end labeling of the primer is more preferable. Furthermore, it is desirable that the sequence or length of the primer is adjusted in consideration of influence of the labeling substance to the Tm value as with the chemical modification group.

In addition, the labeling substance for the amplification product is preferably a fluorescent substance. Alternatively, it may be a radioisotope.

The above-described nucleic acid amplification method according to the present invention is applicable for preparation of a sample nucleic acid to be subjected to a nucleic acid detection analysis based on hybridization. As described above, this method amplifies at least one nucleic acid strand having a specified sequence using at least two primers, and the detection analysis based on hybridization reaction uses DNA molecules immobilized or adsorbed to a solid surface as a probe for hybridization reaction. Such DNA immobilized or adsorbed to a substrate is preferably in a form of DNA chip or DNA micro-array. Alternatively, the substrate may be a bead.

Further, the present invention provides a primer set for exclusive use in PCR amplification method according to the present invention described above, comprising at least two primers used to amplify a nucleic acid strand having a specific sequence.

Also the present invention provides a PCR amplification product obtained by using the nucleic acid amplification method according to the present invention, wherein the amplification product is prepared by using a primer set of two or more primers as described above.

Furthermore, the present invention also provides a nucleic acid detection method based on hybridization reaction between a probe and a sample to be analyzed, where the sample is prepared by using one of the amplification methods of the present invention.

The nucleic acid amplification method according to the present invention will be described more in detail below.

According to the present nucleic acid amplification method, Tm values of the primers (Tm values of the template-primer hybrids) and annealing temperature in PCR are appropriately determined to preferentially amplify a particular nucleic acid strand having a desired base sequence region selected from the all sequence of the template nucleic acid, whereby yields of DNA synthesis from the respective primers of different Tm values will differ to favor synthesis from a specific primer. Each primer is designed so that the Tm value of a primer that is used for extension of a desired single-strand DNA is higher than the Tm values of other primers. In many cases, the base sequence of the primer used for amplification of the DNA strand desired for preferential amplification is designed to show a Tm value higher than the annealing temperature, while the primer used for amplification of the opposite strand is designed to show a Tm value lower than the annealing temperature in PCR, whereby only the desired strand of the template DNA can be amplified preferentially and efficiently.

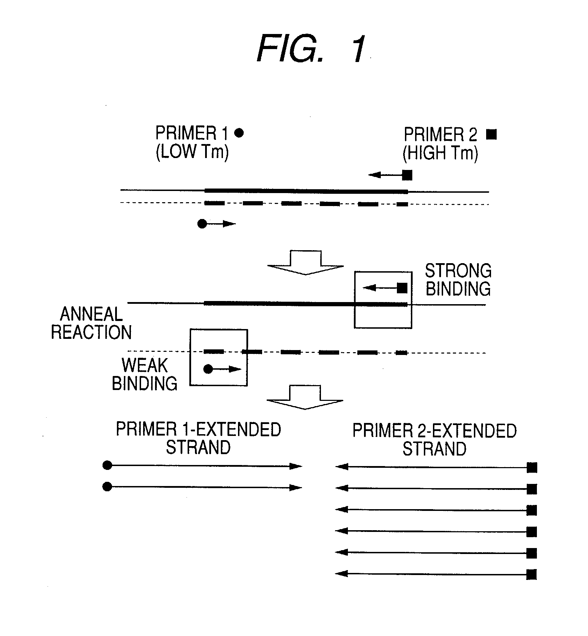

The technical principle of the nucleic amplification method according to the present invention is schematically shown in FIG. 1. In the annealing process, a primer 2 having a higher Tm value binds to the template more strongly, yielding the strand extended from the primer 2 at a higher yield. On the other hand, a primer 1 having a lower Tm value binds to the template more weakly, resulting in lower yield of the primer 1-extended strand. In the PCR amplification reaction, the temperature cycle is repeated to repeat amplification where DNA strands are extended from the corresponding primers using single-stranded DNA molecules synthesized by the preceding stages as templates. According to the present invention, the yield of the primer 1-extended strand is maintained at a certain level by adding the primers in the same amounts. Consequently, it is possible to increase the yield of the desired primer 2-extended strands while avoiding a situation in which amplification substantially stops as a result of drastic decrease in the content of the primer 1-extended strands that serve as the template for primer 2-extended strands in each cycle of amplification.

The PCR amplification product of the method according to the present invention can incorporate a labeling substance such as a fluorescent substance in the process of extension of the DNA strand from the primer. To incorporate the labeling substance to the extended DNA molecules, a label may be attached to the primer in advance, or a labeled nucleotide is used as a substrate in DNA extension by a polymerase enzyme.

When the labeled single-stranded DNA molecules are used as a sample for detection analysis of a nucleic acid molecule based on a hybridization reaction with a DNA probe, the hybrid formed bewteen the probe and the labeled single-strand DNA molecule allows easy quantitative detection. For example, in preparation of a sample to be hybridized with a DNA chip fabricated for detection of a certain nucleic acid, the nucleic acid amplification method according to the present invention can be suitably applied to the sample preparation step using nucleic acid amplification to preferentially and quantitatively amplify the target strand at the same time incorporating a predetermined labeling substance such as a fluorescent substance to the product.

According to the nucleic acid amplification method of the invention, first, primers flanking the region desired to be amplified in a targeted nucleic acid molecule are designed. In designing a set of two primers, a forward primer and a reverse primer flanking the region to be amplified are designed to have different Tm values under PCR conditions. When the Tm value of the primer for extending the DNA strand of which preferential amplification is desired (target strand) is made higher than that of the primer for extension of the other DNA strand, the target strand extended from the primer having a higher Tm value can be amplified in a larger amount.

In order to design the primer so as to have Tm value at a desired temperature, it is common to change the length of the base sequence of the primer without significantly impairing sequence characteristics as the primer, or to select the base sequence of the primer to have different %GC content (composition percentage of guanine and cytosine in the nucleotide sequence) will change. Alternatively, the Tm value of the designed primer can be determined as follows: first the ratio of the primers bound to the template under the actual PCR conditions while changing the temperature, by using, for example, intensity of the fluorescent label attached to the primer, and the Tm value of the primer can be calculated from the dependence of the ratio on the temperature. In addition, there are known methods for estimating Tm value of the primer with high accuracy by calculation on the basis of the base sequence of the primer, such as the nearest neighbor method, the Wallace method and the GC% method.

For the nearest neighbor metnod, see Breslauer K.J., Frank R., Blocker H., Markey L. A. (1986) Predicting DNA duplex stability from the base sequence - Proc. Natl. Acad. Sci. USA 83, 3746-3750, Freier S.M., Kierzek R. Jaeger J. A., Sugimoto N., Caruthers M.H., Nielson T., Turner D.H. (1986) Improved free-energy parameters for predictions of RNA duplex stabilit. - Proc. Natl. Acad. Sci. USA 83, 9373-9377, Schildkraut C., Lifson S. (1965) Dependence of the melting temperature of DNA on salt concentration - Biopolymers 3, 195-208, etc.

For the Wallace method, see Wallace, R.B.;Shaffer, J.; Murphy R.F.; Bonner, J.; Hirose, T.; Itakura, K.; (1979) Nucleic Acid Res. 6, 3543, etc.

For GC% method, see Dependence of the Melting Temperature of DNA on Salt Concentration, Schildkraut C., Lifson S. (1965) BIOPOLYMERS 3.195-208, Optimization of the annealing temperature for DNA amplification in vitro, W. Rychlik, Nucl. Acids Res. (1990) 18(21)6409-6412.

Furthermore, as a method for designing a primer having a desired Tm value under PCR reaction conditions, chemical modification of the primer to adjust the Tm value by the action of the chemical modification group. The chemical modification group bound to the primer influences the double-strand stability. For example, Tm value will increase if the chemical modification group enhances the stability of the double-stranded nucleic acid, while the Tm value will decrease if the chemical modification group reduces the stability.

The chemical modification group may be used without limitation to the type or bonding form. Substances that strongly interact with the double-stranded structure of nucleic acid, such as intercalater or groove binder are especially effective for adjustment of the Tm value. For the intercalater, ethidium bromide, 9-aminoacridine, acridine orange, proflavin, and other polycyclic aromatic molecules such as anthracene are generally used. Groove binders include commercially available HOECHST 33258, HOECHST 33342, Pentamidine, Netropsin and DAPI. Aromatic compounds such as ethidium bromide and anthracene are generally used. In addition, a pyririum pigment disclosed in Japanese Unexamined Patent Publication No. H07-233065, or the like is often used as a substance strongly interacting with the double-stranded nucleic acid. Some groove binders, i.e. DAPI, have been reported to interact with an AT-rich region at a higher frequency. On the other hand, many intercalaters or groove binders strongly interact with the double-stranded nucleic acid, nonspecifically to the base sequence thereof. At this time, the structure and thermal stability of the resultant complex vary depending on the base sequence of the double-strand structure portion of nucleic acid and the type of intercalater or groove binder.

The intercalater enters between a stacking base pair of double-strand nucleic acid, while the groove binder enters a groove of a double-strand helical structure, and both the intercalater and groove binder strongly affect the stability of the double-strand structure, but there are those enhance the stability of the double-stranded structure and those reduce the stability.

Methods for introducing a chemical modification group into the primer include, for example, a method to modify the 5' end of the primer with an amino group or biotin in advance, and then a desired chemical modification group is bonded to the functional group via a reaction specific to such a functional group, but not limited thereto.

The primer set that is used in the basic PCR amplification reaction is comprised of one primer set of a forward primer and a reverse primer flanking a region desired to be amplified, but the nucleic acid amplification method according to the present invention is not limited thereto. The nucleic acid amplification method of the present invention may also be applied in amplification of one region with two or more primer pairs, amplification of a plurality of amplification regions with one primer set, or combined PCR amplification thereof. The nucleic acid amplification method of the present invention can be applied to the PCR amplification reaction using a plurality of primer sets by carrying out a series of selecting steps of selecting one or more nucleic acid molecules desired to be amplified from one or more nucleic acid molecules, selecting one or more regions desired to be amplified from base sequences of the selected nucleic acid molecules, designing primers for the selected regions, and selecting one or more DNA strands desired to be preferentially amplified by the PCR reaction, and then designing primers to be used and setting conditions of the PCR amplification reaction according to the nucleic acid amplification method of the present invention.

The PCR amplification reaction using the designed primers can proceed under any reaction condition, but for effectively carrying out the nucleic acid amplification reaction according to the present invention, the PCR amplification is preferably carried out using an annealing temperature in the PCR amplification reaction set between the different Tm values of two primers, i.e. a set of a forward primer and a reverse primer. With certain PCR amplification reactions, the annealing temperature and the temperature of extension by the polymerase are the same, and thus it is not necessary to carry out the annealing reaction separately. In such a case, the Tm value of each primer is determined in such a manner that the temperature of enzyme reaction and annealing comes between the Tm values of the two primers, whereby preferential nucleic acid amplification by the method of the present invention can be performed.

A relation between the Tm values of two primers and the annealing temperature in the PCR amplification reaction is preferably the relation described above, but the PCR amplification reaction can be carried out even if the annealing temperature is lower than the Tm values of two primers, for example. However, a substantial difference between the Tm values of two primers becomes relatively small compared to the annealing temperature, and thus the effect of the present invention is reduced. Conversely, if the annealing temperature is higher than the Tm values of two primers, the efficiency of the PCR amplification reaction itself may be reduced. Thus, in the amplification method of the present invention, it is most desirable to set the annealing temperature of the PCR amplification reaction to a value between the Tm values of two primers, and if it is impossible to set the annealing temperature to such a value, it is desirable to set the annealing temperature to within ±5°C from the Tm value of one primer.

As long as there is an appropriate difference between the Tm values of two primers under PCR amplification reaction conditions, the nucleic acid amplification method of the present invention can be carried out. Preferably, the difference between the Tm values is in the range of 1 to 5°C. More preferably, the difference between the Tm values is in the range of 5 to 10°C. Further more preferably, the difference between the Tm values is in the range of 10 to 20°C.

In detail, a large difference between the Tm values of two primers is desirable in terms of the object of the invention, i.e. "to amplify the primer-extended strand desired to be preferentially amplified in an amount larger than that of the complementary strand", excessively large difference is not desirable in terms of efficiency of the PCR amplification reaction. Thus, if the asymmetric amplification is required more than amplification rate, a difference in Tm value is set 10 to 20°C or 5 to 10°C for two primers, and if amplification efficiency is required more than asymmetric amplification, the difference in Tm is more preferably set to a relatively low value.

The nucleic acid amplification method according to the present invention is applicable to preparation of various specimens requiring PCR amplification without specific limitation. As a particularly effective embodiment, it is applicable to assay of a nucleic acid molecule having a specific nucleotide sequence using hybridization reaction. Above all, there is a detection method using hybridization to the DNA probes immobilized on a substrate, which has been widely used in recent years as a nucleic acid detection method. For the substrate, glass, plastics, metals and the like are often used, and the nucleic acid amplification method of the present invention can be used irrespective of the type of the substrate. The method to which the present invention is most effectively applicable is a method for assaying nucleic acid molecules using a DNA chip or DNA micro-array where a large number of DNA probes are immobilized on a flat substrate. Furthermore, other than the flat DNA chips, the nucleic acid assay method using beads on which DNA probes are immobilized has become popular in recent years, and the nucleic acid amplification method of the present invention is also applicable to preparation of a sample in the nucleic acid detection method using the DNA probes immobilized on the surfaces of beads.

The DNA probes immobilized on the substrate are single-stranded DNA fragments, and if the strand length of the single-stranded DNA fragment is relatively large, i.e. about 60mer, the Tm value of the single-strand DNA probe and the target strand is relatively high, and thus a relatively strong hybridization reaction can proceed. Therefore, even when the subject PCR amplification product are in a double-stranded structure with its complementary strand, the double-stranded structure can be melted by heating treatment for efficient formation of a hybrid between the probe and a target strand. On the other hand, if the length of the DNA probe on the substrate is relatively short, i.e. about 20mer, the Tm value of the single-strand DNA probe is low, and thus it is impossible to achieve efficient hybridization with the probe and the target strand because of the competition with the annealing of the melted PCR amplification product. When the DNA probe of a relatively small length such as 20mer is used, the nucleic acid amplification method according to the present invention is particularly effective, because the target strand can be preferentially amplified, and highly sensitive detection can be performed on such a sample.

When the nucleic acid amplification method of the invention is carried out in the step of preparing a sample containing a target strand that forms a hybrid with the immobilized DNA probe of the DNA chip, the primer-extended strand desired to be amplified in a large amount, i.e., the target strand, is often labeled. The nucleic acid amplification method according to the present invention is also applicable to the nucleic acid amplification method involving labeling, without restriction by the labeling method. For the method of labeling, the most representative method is to label part of the primer in advance, as described above for chemical modification of the primer, and then carry out the PCR amplification reaction with the labeled primer. Typically, 5' end of the primer or the like is labeled with a fluorescent substance or the like in advance, and this labeled primer is used to carry out the PCR amplification reaction. In this case, however, the primer structure such as base sequence should be designed in consideration of influences of the labeling substance on the Tm value as in the case of the primer using a chemical modification group. Alternatively, PCR substrate deoxynucleotides to be incorporated into the DNA strand may be labeled with a fluorescent substance or the like and added to the PCR reaction solution to label the PCR amplification product. This method is also popular. The nucleic acid amplification method according to the present invention is also applicable to any of these labeling methods without specific limitation.

For the labeling substance, fluorescent substances are generally used because of high sensitivity and easy handling, but it is also possible to use labeling with a radioisotope, and indirect labeling with labeled avidin that binds biotin attached to the PCR amplification product.

### Examples

The present invention will be described more specifically below with Examples. Examples described below represent one example of the best embodiments according to the present invention, but the technical scope of the present invention is not limited to these Examples.

### <Example 1> PCR of pUC118 EcoRI/BAP

### (1) Design of primer

Four primers having the following sequences were designed based on the base sequence of commercially available Vector pUC118 EcoRI/BAP (total length 3162 bp) manufactured by Takara Co., Ltd. The base sequence information of pUC118 EcoRI/BAP is provided by Takara Co., Ltd., and is also available from published databases and the like.

Tm values determined by calculation based on the base sequences of these four primers are also shown in Table 1. The Tm values were calculated using the method described above and the following PCR conditions: Na⁺ concentration in the reaction solution: 50 mM, Mg²⁺ concentration: 1.5 mM, each primer concentration: 0.5 µM.

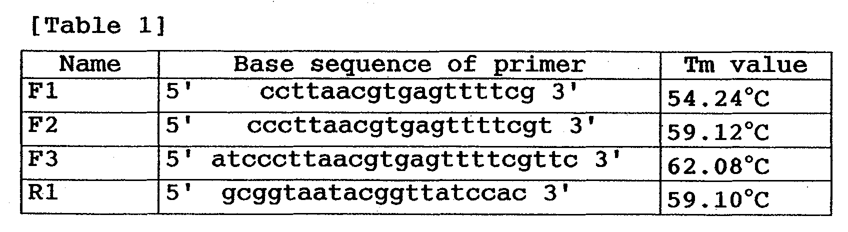

The respective lengths of the double-stranded PCR amplification products obtained using the following combinations of the forward primers and the reverse primer are shown in Table 2.

**[Table 2]**

| Combination of primers | | | Strand length of PCR amplification product |
|---|---|---|---|
| | Forward primer | Reverse primer | |
| [1] | F1 | R1 | 758 bp |
| [2] | F2 | R1 | 759 bp |
| [3] | F3 | R1 | 761 bp |

### (2) Synthesis of primer

Four primers designed in the above (1) were synthesized. Each primer having the designated sequence was synthesized using a DNA synthesizer by a conventional method, and modified by attaching an amino group on the 5' end through a hexamethylene linker. The obtained 5' end amino-modified DNA (primers) were subjected to cartridge purification. F1, F2 and F3 primers were treated with an NHS type Cy3 labeling reagent (Amersham Pharmacia Biotech Inc.) and R1 with an NHS type Cy5 standard reagent (Amersham Pharmacia Biotech Inc.), both bind to amino group, to introduce a fluorescent label to the 5' end. The obtained labeled primers were each subjected to HPLC purification to obtain four primers: Cy3-labeled F1, Cy3-labeled F2, Cy3-labeled F3 and Cy5-labeled R1. The obtained labeled primers were each diluted to a concentration of 10 µM with TE buffer.

### (3) PCR amplification

The PCR amplification was carried out using four labeled primers synthesized in (2), vector pUC118 EcoRI/BAP (manufactured by Takara Co., Ltd.) and a PCR kit HotStarTaq Master Mix (manufactured by QIAGEN Inc.).

With three combinations of forward and reverse primers shown in Table 2, reaction solutions having the composition shown in Table 3 were prepared.

**[Table 3]**

| Composition of reaction solution | |
|---|---|
| Components | Composition |
| Master Mix (Master Mix, QIAGEN Inc.) | 25 µl |
| Template DNA (pUC118 dilution, Takara Co., Ltd.) | 1 µl (10 ng) |
| Cy3-labeled Forward primer (F1, F2 or F3) | 2.5 µl (25 pmol/tube) |
| Cy5-labeled Reverse Primer (R1) | 2.5 µl (25 pmol/tube) |
| H₂O | 19 µl |
| Total | 50 µl |

With each prepared reaction solution, PCR amplification reaction was carried out according to the temperature cycle protocol in Table 4 using a commercial available thermal cycler.

**[Table 4]**

| Temperature conditions of PCR amplification reaction | | | | |
|---|---|---|---|---|
| Step | Temperature | | Retention time | Number of cycles |
| 1 | 95°C | | 15 min. | |
| 2 | 92°C | (denaturation) | 45 s | 25 cycles |
| 3 | 57°C | (annealing) | 45 s | |
| 4 | 72°C | (extension) | 1 min. | |
| 5 | 72°C | | 10 min. | |

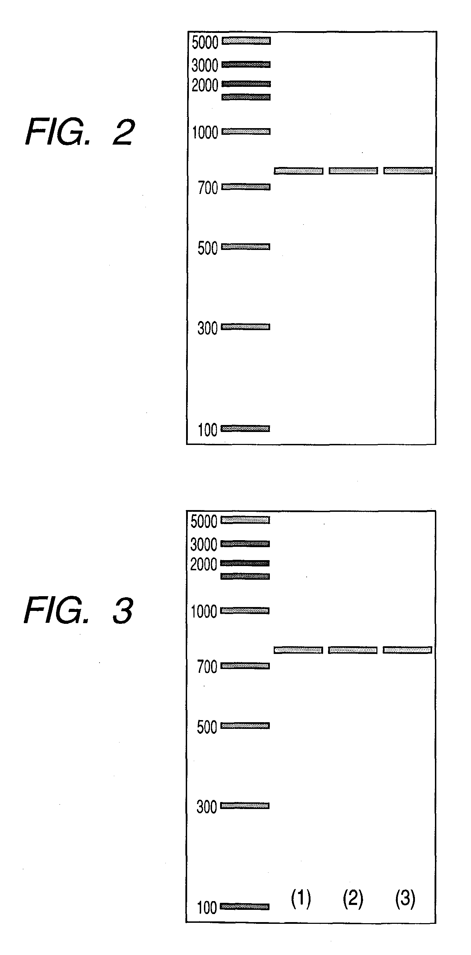

After the amplification reaction was completed, the PCR amplification product was purified using a column for purification (QIAGEN QIAquick PCR Purification Kit). After purification, a solution of the PCR amplification product was prepared in an amount of 50 µl. Part of the resultant purified PCR amplification product solution was taken, and subjected to electrophoresis according to a standar method to confirm that a desired band equivalent to 760 bp appeared. An image of electrophoresis for PCR amplification products obtained by using combinations [1], [2] and [3] of forward and reverse primers is shown in FIG. 2.

### (4) Measurement of absorption

The absorptions of solutions of four labeled primers synthesized in (2) and the PCR amplification product solution prepared in (3) were measured. Furthermore, the labeled primer solutions were each prepared in a concentration of 0.5 µM, and the absorption intensity derived from a labeling fluorescent substance was measured at the absorption wavelength described below depending on the labeling fluorescent substance (Cy3 or Cy5) of each labeled primer.

Furthermore, for each PCR amplification product solution, absorption intensity was measured at the respective absorption wavelengths of the labeling fluorescent substances corresponding to the labeled forward primer and reverse primer.

**[Table 5]**

| Name of primer | Absorption wavelength | Absorption intensity |
|---|---|---|
| Cy3-labeled F1 | 564 nm | 0.0872 |
| Cy3-labeled F2 | 564 nm | 0.0859 |
| Cy3-labeled F3 | 564 nm | 0.0880 |
| Cy5 labeled R1 | 663 nm | 0.1214 |

**[Table 6]**

| PCR amplification product | Absorption wavelength | Absorption intensity |
|---|---|---|
| PCR amplification product [1] | 564 nm (due to Cy3-labeled F1) | 0.0135 |
| | 663 nm (due to Cy5-labeled R1) | 0.0366 |
| PCR amplification product [2] | 564 nm (due to Cy3-labeled F2) | 0.0361 |
| | 663 nm (due to Cy5-labeled R1) | 0.0494 |
| PCR amplification product [3] | 564 nm (due to Cy3-labeled F3) | 0.0605 |
| | 663 nm (due to Cy5-labeled R1) | 0.0752 |

Subsequently, the incorporation yield of each labeled primer by the PCR amplification reaction was calculated based on the measured absorption intensity of the PCR amplification product solution. This incorporation yield indicates the percentage of the primer in the reaction solution participated in the extension reaction to form the PCR amplification product.

**[Table 7]**

| PCR amplification product | Extended strand with labeled primer | Incorporation yield |
|---|---|---|
| PCR amplification product [1] | Forward extension from Cy3-labeled F1 | 15.5% |
| | Reverse extension from Cy5-labeled R1 | 30.1% |
| PCR amplification product [2] | Forward extension from Cy3-labeled F2 | 42.0% |
| | Reverse extension from Cy5-laboled R1 | 40.7% |
| PCR amplification product [3] | Forward extension from Cy3-labeled F3 | 68.8% |
| | Reverse extension due to Cy5-labeled R1 | 61.9% |

As seen from the results, no significant difference in incorporation yield of the Cy3-labeled F2 and Cy5-labeled R1 was observed, indicating even synthesis of corresponding strands, with the PCR amplification product [2] where the both primers had almost the same Tm values and the annealing temperature was below the both Tm values. On the other hand, the PCR amplification product [1] shows preferential incorporation of the Cy5-labeled R1 primer, i.e., preferential synthesis from R1 primer, where the Tm value of Cy5-R1 was higher than that of Cy3-F1 and the annealing temperature was between these Tm values. Also, the PCR amplification product [3] shows preferential incorporation of Cy3-labeled F3 than Cy5-labeled R1, where the Tm value of Cy3-F3 was higher than that of Cy5-R1 although the annealing temperature was lower than these Tm values.

### <Example 2> Hybridization reaction on DNA chip

### (1) Fabrication of DNA micro-chip

### (1)-1 Cleaning of glass substrate

A glass substrate of synthetic quartz (size (WLT): 25 mm × 75 mm × 1 mm, manufactured by Iiyama Tokushu Glass Co., Ltd.) was placed in a heat-resistant and alkali-resistant rack, and immersed in a cleaning liquid prepared at a predetermined concentration for ultrasonic cleaning. The glass substrate was immersed in the cleaning liquid all night, and then ultrasonically cleaned for 20 minutes. Subsequently, the glass substrate was taken out from the liquid, lightly rinsed with pure water, and then ultrasonically cleaned in ultra-pure water for 20 minutes. Then, the glass substrate was immersed in an 1N aqueous sodium hydroxide solution heated to 80°C for 10 minutes. The glass substrate was again rinsed with pure water and ultra-pure water to prepare a cleaned quartz glass substrate for DNA chips.

### (1)-2 Surface treatment

A silane coupling agent KBM-603 (manufactured by Shin-Etsu Silicones) was dissolved in pure water to a concentration of 1%, and stirred at room temperature for 2 hours. Subsequently, the cleaned quartz glass substrate was immersed in the silane coupling agent solution, and left standing at room temperature for 20 minutes. The glass substrate was taken out from the solution, the surface thereof was lightly rinsed with pure water, and nitrogen gas was then blown to the both surfaces of the glass substrate to dry the substrate. Then, the dried glass substrate was baked for 1 hour in an oven heated at 120°C to complete the coupling agent treatment. By this coupling agent treatment, an amino group derived from the silane coupling agent was introduced to the surface of the glass substrate.

On the other hand, an EMCS solution, obtained by dissolving N-(6-maleimidocaproyloxy)succinimide (hereinafter abbreviated as EMCS) (manufactured by Dojindo Laboratories) in a mixed solvent of dimethyl sulfoxide and ethanol (1:1) to a final concentration of 0.3 mg/ml, was prepared. After baking was completed, the treated glass substrate was immersed in the prepared EMCS solution at room temperature for 2 hours. During this immersion treatment, the amino group introduced onto the surface of the glass substrate was reacted with the succinimide group of EMCS to introduce a maleimido group derived from EMCS to the surface of the glass substrate. The glass substrate taken out from the EMCS solution was washed with the mixed solvent of dimethyl sulfoxide and methanol, further washed with ethanol, and then dried under a nitrogen gas atmosphere.

### (1)-3 Synthesis of DNA for probes

Probe P1 having the following base sequence was synthesized. A portion fully complementary to P1 is present at or near the 3' end portion of the reverse strand, a strand extended from the reverse primer, of the PCR amplification products [1], [2] and [3] in Example 1.

Probe DNA was thiolated at the 5' end according to a standard method for enabling covalent bonding to the glass substrate having maleimido groups on the surface. Thereafter, protecting groups introduced to avoid a side reaction during DNA synthesis were removed, and the prove DNA was subjected to HPLC purification and a desalination treatment.

The resultant probe DNA was dissolved in pure water, and the resultant solution was divided so that each would have a final concentration (when dissolved in ink) of 10 µM, and then freeze-dried to remove water.

### (1)-4 Discharge of probe DNA by BJ printer and binding of probe DNA to substrate surface

An aqueous solution containing 7.5 wt% of glycerin, 7.5 wt% of thioglycol, 7.5 wt% of urea and 1.0 wt% of Acetylenol EH (manufactured by Kawaken Fine Chemicals Co., Ltd.) was prepared. Subsequently, the divided probe DNA was dissolved in the mixed solvent so that it had a specified concentration (10 µM). The resultant probe DNA solution was filled in an ink tank for Bubble Jet Printer (trade name: BJF-850, manufactured by Canon Inc.), and the ink tank was mounted on a printing head.

Furthermore, the Bubble Jet Printer is a printer modified so as to allow printing on a flat plate. Furthermore, in the modified Bubble Jet Printer, about 5 pl of droplet of DNA solution can be spotted at about 120 µm pitches by imputing a print pattern according to a specified file creation process.

Subsequently, this modified Bubble Jet printer was used to spot the probe DNA solution on the surface of the glass substrate. A pattern of printing was created in advance so that 16 spots would be discharged per DNA chip, and ink jet printing was performed. After ensuring that the DNA solution was reliably spotted in the desired pattern by a magnifier or the like, and then the glass substrate was left standing in a humidified chamber for 30 minutes to make the maleimido group on the surface of the glass substrate react with a sulfanil group (-SH) at the probe DNA 5' end.

### (1)-5 Cleaning

After the reaction proceeded in the humidified chamber for 30 minutes, unreacted probe DNA remaining on the surface of the glass substrate was washed away with a 10 mM phosphate buffer solution (pH 7.0) containing 100 mM of NaCl. A DAN micro-array DNA chip with specified single-strand probe DNA immobilized on the surface of the glass substrate in 16 spots per DNA chip was obtained.

### (2) Hybridization reaction

The DNA chip fabricated in Example 2 (1) and the PCR amplification products fabricated in Example 1 as a specimen were used to carry out a detection reaction for a target nucleic acid molecule.

### (2)-1 Blocking of DNA micro-array

BSA (bovine serum albumin Fraction V: manufactured by Sigma Co., Ltd.) was dissolved to 1 wt% in a 100 mM NaCl/10 mM phosphate buffered saline, and the DNA micro-array (DNA chip) fabricated in Example 2 (1) was immersed in this solution at room temperature for 2 hours to perform blocking of the surface of the grass substrate. After blocking was completed, the DNA micro-array was washed with a 2SSC solution (NaCl 300 mM, sodium citrate (trisodium citrate dihydrate, C₆H₅Na₃·2H₂O) 30 mM, pH 7.0) containing 0.1 wt% SDS (sodium dodecyl sulfate), and then rinsed with pure water. Thereafter, the DNA micro-array was drained by a spin-drying apparatus.

### (2)-2 Preparation of hybridization solution

The concentrations of three kinds of PCR amplification products were adjusted so that the intensities of absorption by Cy5 with which the reverse strand was labeled were the same in order to equalize the amounts (mol numbers) of target nucleic acid in the PCR amplification product solutions. Subsequently, three kinds of hybridization solutions were prepared using equal parts of PCR amplification product solutions so that the final concentration would match the composition described below.

### Hybridization solution

6SSPE/10% Formamide/PCR amplification product solution (6xSSPE: NaCl 900 mM, NaH₂PO₄·H₂O 60 mM, EDTA 6mM, pH 7.4)

### (2)-3 Hybridization

The drained DNA chip was set in a hybridization apparatus (Genomic Solutions Inc. Hybridization Station), and the hybridization solution having the composition described above was used to carry out a hybridization reaction according to the procedure described below.

### Hybridization conditions and procedure

**[Table 8]**

| Operation | Operation procedure/conditions |
|---|---|
| Reaction | 65°C 3 min → 92°C 2 min → 45°C 3 h |
| Cleaning | 2×SSC/0.1% SDS at 25°C |
| | 2×SSC at 20°C |
| (Rinse) | H₂O (Manual rinsing) |
| Drying | Spin dry |

### (3) Measurement of fluorescence

After the hybridization reaction was completed, a fluorescence detecting apparatus for DNA micro-arrays (Genepix 4000B manufactured by Axon Instruments, Inc.) was used to measurement a fluorescence derived from a hybrid for the spin-dried DNA chip. The fluorescence derived from the hybrid is derived from fluorescent labels on strands on the reverse side of PCR amplification products [1], [2] and [3] in Example 1, which form the hybrid with probe DNA.

The fluorescence intensity observed in a spotless portion of probe DNA on the DNA chip was determined to be a background value, and a value obtained by subtracting the background value from the apparent fluorescence intensity from each spot was determined to be a measured value of fluorescence intensity. Furthermore, the average value of measured values obtained by making the measurement twice is shown in Table 9 below.

**[Table 9]**

| PCR amplification products of PCR specimen | Fluorescence intensity |
|---|---|
| PCR amplification product [1] | 7400 |
| PCR amplification product [2] | 3250 |
| PCR amplification product [3] | 2670 |

From the result, it can be understood that more efficient hybridization occurred on the DNA chip with the PCR amplification product [1] where the reverse strand had been synthesized in preference to the forward strand, than with the PCR amplification product [2] where the forward strand and the reverse strand had been equally synthesized. Thus, it was shown that by applying the nucleic acid amplification method according to the present invention, a desired PCR amplification extended strand could be synthesized more preferentially, and the nucleic acid amplification method according to the present invention could suitably be applied to a step of preparing a sample for use in detection of a nucleic acid molecule using a DNA chip.

### <Example 3> PCR and hybridization using anthracene-modified primer

### (1) Synthesis of anthracene-modified primer

Primer R1 of pUC118 designed in Example 1 was synthesized. The primer molecules were synthesized by using an automatic DNA synthesizer according to a standard method, to each of which a thiol group was attached at the 5' end via a methylene chain linker. The obtained 5' end thiol-modified DNA was purified by gel filtration and ethanol precipitation after a protecting group bound to each functional group was removed.

Subsequently, 30 nmol of obtained thiol-modified DNA was dissolved in 100 microliters of mixed solvent (50% of water, 25% of ethanol and 25% of dimethyl sulfoxide), 1 mg of EMCS was added to the resultant solution and reacted at room temperature for 3 hours. The resultant mixture was purified by gel filtration and ethanol precipitation. As a result of the reaction, EMCS bound to the 5' end of the DNA to provide a modified DNA having a succinimide group at the end.

Subsequently, the modified DNA was dissolved in 100 microliters of mixed solvent of water and dimethyl formamide (1:1), then 10 mg of 2-aminoanthracene was added to the resultant solution, and the resultant mixture was stirred to dissolve it as much as possible, and reacted at 45°C for about 5 hours with stirring. As a result, the succinimide group at the DNA end reacted with the amino group of aminoanthracene to give a modified primer having anthracene at the 5' end (hereinafter abbreviated as R1AN).

After precipitation was removed by filtration, the primer was purified according to a standard method, and the concentration of the nucleic acid component was determined. Then the primer was diluted to a concentration of 10 µM with TE buffer.

### (2) PCR amplification reaction

Primers having the sequences of F2 and R1 designed in Example 1 were synthesized without modifying the end. The synthesized primer was diluted to a concentration of 10 µM with TE buffer.

Subsequently, three primers, i.e. F2, R1 and R1AN, were used to carry out the PCR. Combinations of primers are shown in Table 10.

**[Table 10]**

| Combination of primers | | | Strand length of PCR amplification product |
|---|---|---|---|
| | Forward primer | Reverse primer | |
| [4] | F2 | R1AN | 759 bp |
| [5] | F2 | R1 | 759 bp |

The detailed reaction composition of the PCR is shown in Table 11 below. The PCR was carried out mixing Cy3dUTP into the substrates to label the PCR product.

**[Table 11]**

| Composition of reaction solution | |
|---|---|
| Components | Composition |
| Master Mix (Master Mix, QIAGEN Inc.) | 25 µl |
| Template DNA (pUC118 dilution, Takara Co., Ltd.) | 1 µl (10 ng) |
| Forward primer (F2) | 2.5 µl (25 pmol/tube) |
| Reverse primer (R1AN or R1) | 2.5 µl (25 pmol/tube) |
| Cy3dUTP (manufactured by Amersham Pharmacia Biotech Inc.) 1 mM | 2.5 µl (25 nmol/tube) |
| H₂O | 16.5 µl |
| Total | 50 µl |

With the prepared reaction solution, PCR amplification reaction was carried out according to the temperature cycle protocol in Table 12 below using a commercially available thermal cycler.

**[Table 12]**

| Temperature conditions of PCR amplification reaction | | | | |
|---|---|---|---|---|
| Step | Temperature | | Retention time | Number of cycles |
| 1 | 95°C | | 15 min. | |
| 2 | 92°C | (denaturation) | 45 s | 25 cycles |
| 3 | 57°C | (annealing) | 45 s | |
| 4 | 72°C | (extension) | 1 min. | |
| 5 | 72°C | | 10 min. | |

After the amplification reaction was completed, the PCR amplification product was purified using a column for purification (QIAGEN QIAquick PCR Purification Kit). After purification, a solution of the PCR amplification product was prepared in an amount of 50 µl. Part of the resultant purified PCR amplification product solution was taken, and subjected to electrophoresis according to a standard method to confirm the presence of a desired band equivalent to about 760 bp.

### (3) Hybridization reaction

All of the collected two kinds of PCR products were hybridized with the DNA chips fabricated in Example 2 (1) respectively. The composition of the hybridization solution, the procedure of hybridization and the scan of the chip were the same as those in Example 2. The average value for two scans calculated in the same manner as in Example 2 is shown in Table 13.

**[Table 13]**

| PCR amplification products of specimen | Fluorescence intensity |
|---|---|
| PCR amplification product [4] | 10100 |
| PCR amplification product [5] | 7150 |

From the result, it can be understood that primer R1AN having a higher Tm value due to the bound anthracene as an intercalater caused preferential extension reaction than the primer F2. As a result, use of primer R1AN ([4]) increased the fluorescence intensity compared with use of unmodified primer R1 ([5]).

### <Example 4> PCR and hybridization using adamantane-modified primer

### (1) Synthesis of adamantane-modified primer

Primer R1 of pUC118 designed in Example 1 was synthesized by using an automatic DNA synthesizer according to a standard method, to each of which a thiol group was attached at the 5' end via a methylene chain linker. The obtained 5' end thiol-modified DNA was purified by gel filtration and ethanol precipitation after a protecting group bound to each functional group was removed.

Subsequently, 30 nmol of obtained thiol-modified DNA was dissolved in 100 microliters of mixed solvent (50% of water, 25% of ethanol and 25% of dimethyl sulfoxide), 1 mg of EMCS was added to the resultant solution and reacted at room temperature for 3 hours. The resultant mixture was purified by gel filtration and ethanol precipitation. As a result of the reaction, EMCS bound to the 5' end of the DNA to provide a modified DNA having a succinimide group at the end.

Subsequently, the modified DNA was dissolved in 100 microliters of water, then 10 mg of amantadine HCl was added to the resultant solution, and the resultant mixture was stirred to dissolve it as much as possible, and reacted at 45°C for about 5 hours with stirring. As a result, the succinimide group at the DNA end reacted with the amino group of amantadine to give a modified primer having adamantane at the 5' end (hereinafter abbreviated as R1AD).

After precipitation was removed by filtration, the primer was purified according to a standard method, and the concentration of the nucleic acid component was determined. Then the primer was diluted to a concentration of 10 µM with TE buffer.

### (2) PCR amplification reaction

F2 and R1 primers having the sequences designed in Example 1 were synthesized without end modification. Eeach synthesized primer was diluted to a concentration of 10 µM with TE buffer.

Subsequently, three primers, i.e. F2, R1 and R1AD were used to carry out PCR. Combinations of primers are shown in Table 14.

**[Table 14]**

| Combination of primers | | | Strand length of PCR amplification product |
|---|---|---|---|
| | Forward primer | Reverse primer | |
| [6] | F2 | R1AD | 759 bp |
| [7] | F2 | R1 | 759 bp |

The detailed reaction composition of the PCR is shown in Table 15 below. The PCR was carried out adding Cy3dUTP to the reaction substrates to label the PCR product.

**[Table 15]**

| Composition of reaction solution | |
|---|---|
| Components | Composition |
| Master Mix (Master Mix, QIAGEN Inc.) | 25 µl |
| Template DNA (pUC118 dilution, Takara Co., Ltd.) | 1 µl (10 ng) |
| Forward primer (F2) | 2.5 µl (25 pmol/tube) |
| Reverse primer (R1AD or R1) | 2.5 µl (25 pmol/tube) |
| Cy3dUTP (manufactured by Amersham Pharmacia Biotech Inc.) 1 mM | 2.5 µl (25 nmol/tube) |
| H₂O | 16.5 µl |
| Total | 50 µl |

For the prepared reaction solution, the PCR amplification reaction was carried out according to the temperature cycle protocol in Table 16 below using a commercially available thermal cycler.

**[Table 16]**

| Temperature conditions of PCR amplification reaction | | | | |
|---|---|---|---|---|
| Step | Temperature | | Retention time | Number of cycles |
| 1 | 95°C | | 15 min. | |
| 2 | 92°C | (denaturation) | 45 s | 25 cycles |
| 3 | 57°C | (annealing) | 45 s | |
| 4 | 72°C | (extension) | 1 min. | |
| 4 | 72°C | | 10 min. | |

After the amplification reaction was completed, the PCR amplification product was purified using a column for purification (QIAGEN QIAquick PCR Purification Kit). After purification, the PCR amplification product solution was prepared in an amount of 50 µl. Part of the resultant purified PCR amplification product solution was taken, and subjected to electrophoresis according to a standard method to confirm presence of a desired band corresponding to about 760 bp.

### (3) Hybridization reaction

Two kinds of PCR products obtained were each hybridized with the DNA chip fabricated in Example 2 (1) using its total collected amount. The composition of the hybridization solution, the procedure of hybridization and the scan of the chip were the same as in Example 2. The average value for two scans calculated in the same manner as in Example 2 is shown in Table 17.

**[Table 17]**

| PCR amplification products of specimen | Fluorescence intensity |
|---|---|
| PCR amplification product [6] | 5300 |
| PCR amplification product [7] | 9900 |

From the result, it can be understood that primer R1AD chemically modified with adamantane had reduced double-strand stability due to the effect of adamantane, its Tm value thus decreased, and the extension reaction of PCR proceeded in preference for the extended strand from primer F2 relatively. As a result, use of primer R1AD ([6]) decreased the fluorescence intensity compared with use of unmodified primer R1 ([7]).

### Example 5> Two-stage PCR method

### (1) Synthesis of primer

Four primers (R1, F1, F2 and F3) were synthesized and labeled to obtain four primers: Cy3-labeled F1, Cy3-labeled F2, Cy3-labeled F3 and Cy5-labeled R1, in the same manner as in Example 1 (2).

### (2) Amplification of target single-strand nucleic acid

The PCR reaction was carried out using the four primers described above, Vector pUC118 EcoRI/BAP manufactured by Takara Co., Ltd. and PCR Kit Premix Tag (Takara EX Tag Version) manufactured by Takara Co., Ltd.

Three combinations of primers described in (1) were used for PCR of the following conditions and protocols.

| Reaction solution | |
|---|---|
| Premix Taq (Takara EX Taq Version) | 25 µl |
| Template DNA (pUC118 dilution, Takara Co., Ltd.) | 1 µl (2 ng) |
| Cy3-labeled forward primer (F1, F2 or F3) | 3 µl |
| Cy5-labeled reverse primer (R1) | 3 µl |
| H₂O | 18 µl |
| Total | 50 µl |

The reaction solution was subjected to PCR reaction by using a commercially available thermal cycler according the following temperature cycle protocol:
92°C/2 minutes of retention,
STAGE I: 15 cycles of 92°C (denaturation)/45 seconds, 52°C (annealing)/45 seconds and 72°C (extension)/1 minute,
STAGE II: 25 cycles of [92°C (denaturation)/45 seconds, 58°C (annealing)/45 seconds and 72°C (extension)/1 minute], and
retention at 72°C for 10 minutes.

After the reaction was completed, the reaction product was purified using a column for purification (QIAGEN QIAquick PCR Purification Kit). The solution after purification was prepared in an amount of 50 µl. Part of the resultant PCR product was taken, and subjected to electrophoresis according to a standard method to confirm presence of a desired band corresponding to 760 bp. The electrophoresis pattern is shown in FIG. 3. Furthermore, PCR products obtained from the above primer combinations 1' to 3' are called PCR products 1' to 3' respectively.

### (3) Measurement of absorption

Absorption of primers synthesized in (1) and the PCR product synthesized in (2) were measured. The labeled primers were each prepared in a concentration of 0.6 µM, and the absorption intensity was measured at the absorption wavelength described below depending on the labeling fluorescent substance (Cy3 or Cy5). Furthermore, for the PCR product, the absorption intensity at both wavelengths were measured.

**[Table 19]**

| Name of primer | Wavelength | Absorption intensity |
|---|---|---|
| Cy3-labeled F1 | 564 nm | 0.108 |
| Cy3-labeled F2 | 564 nm | 0.103 |
| Cy3-labeled F3 | 564 nm | 0.110 |
| Cy5 labeled R1 | 663 nm | 0.147 |

**[Table 20]**

| Name of PCR product | Wavelength | Absorption intensity |
|---|---|---|
| PCR product 1' | 564 nm (due to Cy3-labeled F1) | 0.0112 |
| | 663 nm (due to Cy5-labeled R1) | 0.0343 |
| PCR product 2' | 564 nm (due to Cy3-labeled F2) | 0.0342 |
| | 663 nm (due to Cy5-labeled R1) | 0.0515 |
| PCR product 3' | 564 nm (due to Cy3-labeled F3) | 0.0609 |
| | 663 nm (due to Cy5-labeled R1) | 0.0763 |

Subsequently, the incorporation yield by the PCR reaction was calculated from the measured absorption intensity. By calculating this incorporation yield, the percentage of the primer used in the extension reaction as the PCR product, of the primer supplied for the PCR reaction, can be determined.

**[Table 21]**

| Name of PCR product | Wavelength | Incorporation yield |
|---|---|---|
| PCR product 1' | 564 nm (due to Cy3-labeled F1) | 10.4% |
| | 663 nm (due to Cy5-labeled R1) | 23.3% |
| PCR product 2' | 564 nm (due to Cy3-labeled F2) | 33.2% |
| | 663 nm (due to Cy5-labeled R1) | 35.0% |
| PCR product 3' | 564 nm (due to Cy3-labeled F3) | 55.4% |
| | 663 nm (due to Cy5-labeled R1) | 51.9% |

For the PCR product 2' in which the Tm value of Cy3-labeled F2 was almost equal to the Tm value of Cy5-labeled R1, it was shown that there was no significant difference in incorporation yield, and thus double strands were equally synthesized. On the other hand, for the PCR product 1', because the Tm value of Cy3-labeled F1 was low, the extended matter from Cy5-labeled R1 was preferentially synthesized, and the incorporation yield of the Cy5-labeled R1 extended matter was much larger than the incorporation yield of the Cy3-labeled F1 extended matter. Furthermore, for the PCR product 3', because the Tm value of Cy3-labeled F3 was higher than the Tm value of Cy5-labeled R1, the extended strand from Cy3-labeled F3 was preferentially synthesized, and had a high incorporation yield.

### <Example 6> Comparison between presence and absence of pre-amplification step

### (1) Amplification of target single-strand nucleic acid

The PCR reaction was carried out using four primers synthesized in Example 5 (1), Vector pUC118 EcoRI/BAP manufactured by Takara Co., Ltd., and PCR kit Premix Taq (Takara EX Taq Version) manufactured by Takara Co., Ltd. Combinations of primers were the same as in Example 5.

| Reaction solution | |
|---|---|
| Premix Taq (Takara EX Taq Version) | 25 µl |
| Template DNA (pUC118 dilution, Takara Co., Ltd.) | 1 µl (2 ng) |
| Cy3-labeled forward primer (F1, F2 or F3) | 3 µl |
| Cy5-labeled reverse primer (R1) | 3 µl |
| H₂O | 18 µl |
| Total | 50 µl |

The reaction solution having the composition described above was subjected to PCR amplification by using a commercially available thermal cycler according the following temperature cycle protocol:
92°C/15 minutes of retention;
40 cycles of 92°C (modification)/45 seconds, 57°C (annealing)/45 seconds followed by 72°C (extension)/1 minute; and
72°C/10 minutes of retention.

After the reaction was completed, the reaction product was purified using a column for purification (QIAGEN QIAquick PCR Purification Kit). The solution after purification was prepared in an amount of 50 µl. Part of the resultant PCR product was taken, and subjected to electrophoresis in the same manner as in Example 5 to confirm presence of a desired band corresponding to 760 bp (not shown).

### (2) Measurement of absorption

The absorption of the PCR product synthesized in (1) was measured. Labeled primers prepared in Example 5 (1) were used. For the PCR product, the absorption intensity at two wavelengths corresponding to respective labeling agents were measured in the same manner as in Example 5.

**[Table 22]**

| Name of primer | Wavelength | Absorption intensity |
|---|---|---|
| Cy3-labeled F1 | 564 nm | 0.108 |
| Cy3-labeled F2 | 564 nm | 0.103 |
| Cy3-labeled F3 | 564 nm | 0.110 |
| Cy5 labeled R1 | 663 nm | 0.147 |

**[Table 23]**

| Name of PCR product | Wavelength | Absorption intensity |
|---|---|---|
| PCR product 1' | 564 nm (due to Cy3-labeled F1) | 0.0066 |
| | 663 nm (due to Cy5-labeled R1) | 0.0144 |
| PCR product 2' | 564 nm (due to Cy3-labeled F2) | 0.015 |
| | 663 nm (due to Cy5-labeled R1) | 0.0219 |
| PCR product 3' | 564 nm (due to Cy3-labeled F3) | 0.0246 |
| | 663 nm (due to Cy5-labeled R1) | 0.0304 |

Subsequently, the incorporation yield by the PCR reaction was calculated from the measured absorption intensity. By calculating this incorporation yield, the percentage of primer used in the extension reaction as the PCR product, of the primer supplied for the PCR reaction, can be determined.

**[Table 24]**

| Name of PCR product | Wavelength | Incorporation yield |
|---|---|---|
| PCR product 1' | 564 nm (due to Cy3-labeled F1) | 6.1% |
| | 663 nm (due to Cy5-labeled R1) | 9.8% |
| PCR product 2' | 564 nm (due to Cy3-labeled F2) | 14.6% |
| | 663 nm (due to Cy5-labeled R1) | 14.9% |
| PCR product 3' | 564 nm (due to Cy3-labeled F3) | 22.4% |
| | 663 nm (due to Cy5-labeled R1) | 20.7% |

Product ratios (F1/R1, F2/R1, F3/R1) derived from the primers are not significantly different from those in Example 5, but the synthesis amount of each product is larger in Example 5. Comparison with Example 5 confirms effectiveness of the pre-amplification step where annealing is conducted at a temperature lower than the lowest Tm value when the amount of DNA as a template is small.

### <Example 7> Hybridization on DNA chip

The detection reaction was carried out in the same manner as in Example 2 (2), using the DNA chip fabricated in Example 2 (1) and the PCR products produced in Example 5.

Preparation of the hybridization solution and hybridization conditions were the same as in Example 2 (2).

### (3) Measurement of fluorescence

A fluorescence detection apparatus for DNA micro-arrays (GenePix 4000B manufactured by Axon Instruments, Inc.) was used to make measurements of fluorescence for the DNA chip after completion of the hybridization reaction (photomultiplier voltage: 400 V, wavelength: 532 nm). Measurement was carried out twice and the obtained data were averaged subtracting the background intensity (fluorescence from the portion without probe spots). The results are shown in Table 25.

**[Table 25]**

| PCR product | Fluorescence intensity |
|---|---|
| PCR product 1' | 5120 |
| PCR product 2' | 2770 |
| PCR product 3' | 2580 |

The result showed that the PCR product 1' containing more reverse strands hybridized more efficiently to the DNA chip than the PCR product 2' containing the forward strands and the reverse strands equally. Thus, the nucleic acid amplification method of the present invention enables efficient synthesis of a desired strand in PCR, and applicable to preparation of a sample for the DNA chip analysis.

The present invention can be applied to preparation of a sample having the increased content of desired single-strand nucleic acid using the PCR method and particularly, the present invention can be suitably applied to detection of a nucleic acid molecule having a specific nucleotide sequence by hybridization reaction by utilizing its effect of increasing the content of desired single-strand nucleic acid.

The present invention is not limited to the above embodiments and various changes and modifications can be made within the spirit and scope of the present invention. Therefore to apprise the public of the scope of the present invention, the following claims are made.

## Claims

1. A method for producing an amplification product having a specific base sequence from a nucleic acid molecule in a sample by PCR amplification using a primer set comprised of at least two primers,
wherein at least one primer of the primer set has a melting temperature (Tm) different from the other primer or primers under conditions of the PCR amplification.

2. The method according to claim 1, wherein a primer having a Tm value higher than the other primer or primers is used for extension of a strand to which most preferential amplification is desired.

3. The method according to claim 2, wherein said sample contains plural kinds of nucleic acid molecules, the method comprising the steps of:
selecting at least one of the nucleic acid molecules desired to be amplified from said plurality of kinds of nucleic acid molecule contained in said sample;
selecting at least one base sequence region desired to be amplified from the entire base sequence of the selected nucleic acid molecule desired to be amplified;
designing a forward primer and a reverse primer for PCR amplification for each of the selected base sequence region or regions as the primer set; and
selecting a strand to which most preferential amplification is desired; and
carrying out PCR amplification with the primers of the primer set and the nucleic acids in the sample.

4. The method according to claim 2, wherein two primers are used for amplification of the product having a specific base sequence, and a primer having a Tm value higher than the other primer is used for extending the strand to which most preferential amplification is desired.

5. The method according to claim 1, wherein an annealing temperature for binding the primers to a template in the PCR amplification is set within a temperature range between the highest and lowest Tm values of the primers constituting the primer set under conditions of the PCR amplification.

6. The method according to claim 1, wherein the PCR amplification comprises at least two annealing steps at different temperatures, the annealing steps consisting of:
a first annealing step at a temperature equal to or less than the lowest Tm value of the plurality of primers constituting said primer set; and
a second annealing step at a temperature in a range between the highest and lowest Tm values of the primers constituting said primer set.

7. The method according to claim 6, wherein the primer set consists of two primers, and the second annealing step is carried out at a temperature in a range between the Tm values of these primers under conditions of said PCR amplification.

8. The method according to claim 4 or claim 6, wherein in difference in temperature between the Tm values of the two primers is in the range of 10°C to 20°C.

9. The method according to claim 4 or claim 6, wherein the difference in temperature between the Tm values of the two primers is controlled in the range of 5°C to 10°C.

10. The method according to claim 4 or claim 6, wherein the difference in temperature between the Tm values of the two primers is controlled in the range of 1°C to 5°C.

11. The method according to any preceding claim, wherein means for controlling at least one primer of the plurality of primers constituting said primer set so as to have a Tm value different from those of the other primers under conditions of said PCR amplification is means for controlling GC% in the base sequence of the primer so as to control the Tm value exhibited by the primer having said base sequence, so that a difference is provided in Tm value between the primer and the other primers by selection of the base sequence of the primer.

12. The method according to any one of claims 1 to 10, wherein means for controlling at least one primer of the plurality of primers constituting said primer set so as to have a Tm values different from those of the other primers under conditions of said PCR amplification reaction is means for controlling the base strand length of the base sequence of the primer so as to control the Tm value exhibited by the primer having said base sequence, so that a difference is provided in Tm value between the primer and the other primers by selection of the base sequence of the primer.

13. The method according to any one of claims 1 to 10, wherein means for controlling at least one primer of the plurality of primers constituting said primer set so as to have a Tm value different from those of the other primers under conditions of said PCR amplification reaction, is chemical modification of the primer.

14. The method according to claim 13, wherein the chemical modification is a chemical substance having a property of enhancing double-strand stability of nucleic acid.

15. The method according to claim 13, wherein the chemical modification is a chemical substance having a property of reducing double-strand stability of nucleic acid.

16. The method according to claim 14, wherein the chemical substance has a property of an intercalater for nucleic acid.

17. The method according to claim 14, wherein the chemical substance has a property of a groove binder.

18. The method according to any preceding claim, further comprising a step of intercomparing the Tm values of at least two primers, which are among the plurality of primers constituting said primer set, under conditions of said PCR amplification reaction,
wherein values calculated based on the base sequences of the primers are used as the Tm values of the primers to be compared, and
the nearest neighbor method is used as a method for calculating the Tm values of the primers.

19. The method according to any one of claims 1 to 17, further comprising a step of intercomparing the Tm values of at least two primers, which are among the plurality of primers constituting said primer set, under conditions of said PCR amplification reaction,
wherein values calculated based on the base sequences of the primers are used as the Tm values of the primers to be compared, and
the Wallace method is used as a method for calculating the Tm values of the primers.

20. The method according to any one of claims 1 to 17, further comprising a step of intercomparing the Tm values of at least two primers, which are among the plurality of primers constituting said primer set, under conditions of said PCR amplification reaction,
wherein values calculated based on the base sequences of the primer are used as the Tm values of the primers to be compared, and
the GC% method is used as a method for calculating the Tm values of the primers.

21. The method according to any preceding claim, wherein the amplification product obtained by PCR amplification includes an amplification product labeled with a labeling substance.

22. The method according to claim 21, wherein the amplification product labeled with said labeling substance is an amplification product such that a labeled deoxynucleotide is incorporated in a deoxynucleotide for use as a substrate in the PCR amplification reaction so that the amplification product has a label derived from said labeled deoxynucleotide in strands extended by the PCR amplification reaction.

23. The method according to claim 21, wherein the amplification product labeled with said labeling substance is an amplification product such that a previously labeled primer(s) is incorporated in the plurality of primers constituting said primer set, and the amplification product is formed as an extended strand of said labeled primer through the PCR amplification reaction.

24. The method according to claim 23, wherein in the previously labeled primer contained, the label previously given to the primer is a 5' end label.

25. The method according to any one of claims 21 to 24, wherein in the amplification product labeled with said labeling substance, the labeling substance is a fluorescent substance.

26. The method according to any one of claims 21 to 24, wherein in the amplification product labeled with said labeling substance, the labeling substance is a radioisotope.

27. The method according to any preceding claim, wherein said sample is a sample to be subjected to detection of nucleic acid molecules using the hybridization reaction,
at least one nucleic acid molecules detected in detection of nucleic acid molecules using said hybridization reaction is an amplification product having at least one specific base sequence, produced by the PCR amplification method using said primer set constituted by at least two primers, and
the sample containing the amplification product prepared by amplification is used as a specimen in detection of nucleic acid molecules using said hybridization reaction.

28. The method according to claim 27, wherein in the detection of nucleic acid molecules using said hybridization reaction, DNA molecules immobilized or adsorbed to a substrate surface are used as probes for the hybridization reaction.

29. The method according to claim 28, wherein in the DNA molecule immobilized or adsorbed to said substrate surface, said substrate has a form of a DNA chip or DNA micro-array.

30. The method according to claim 28, wherein in the DNA molecule immobilized or adsorbed to said substrate surface, said substrate is a bead.

31. A primer set comprising at least two primers, wherein the primer set is used to produce an amplification product having at least one specific base sequence of a nucleic acid molecule from the nucleic acid molecule contained in a sample by the PCR amplification method, and
the primer set constituted by a plurality of primers applicable to the step of producing said amplification product by the PCR method using the nucleic acid amplification method according to any one of claims 1 to 30.

32. A PCR amplification product having at least one specific base sequence of a nucleic acid molecule, produced from the nucleic acid molecule contained in a sample by the PCR amplification method using a primer set of at least two primers,
wherein said amplification product is prepared by the nucleic acid amplification method according to any one of claims 1 to 30.

33. A method for detecting a nucleic acid molecule using a hybridization reaction,
wherein an amplification product having at least one specific base sequences of a nucleic acid molecule is produced from a sample containing the nucleic acid molecule to be detected by the PCR amplification method using a primer set constituted by at least two primers,
a sample containing the amplification product prepared by amplification is used as a specimen in detection of the nucleic acid molecule using said hybridization reaction, and
the nucleic acid amplification method according to any one of claims 1 to 30 is used when said amplification product is produced by the PCR amplification method.

34. A method for amplifying at least one specific nucleotide sequence of a nucleic acid contained in a sample by a PCR method using a primer set of at least two primers, wherein one primer of the primer set for extending a strand having a specific nucleotide sequence has a Tm value different from the other primer or primers under annealing conditions of PCR, the amplification method comprising the steps of:
carrying out the PCR having an annealing step at a temperature equal to or less than the Tm values of primers constituting the primer set in a reaction solution containing said sample and said primer set to obtain a reaction solution having amplified fragments containing said specific nucleotide sequence; and
carrying out the PCR having an annealing step at a temperature equal to or less than the Tm value of the primer for extending the fragment having the specific nucleotide sequence and higher than the Tm values of the other primers, which are among primers constituting the primer set in the reaction solution, for the reaction solution having amplified fragments having said specific nucleotide sequence, to further amplify the fragment having the specific nucleotide sequence.


### SEQUENCE LISTING

<110> Canon Kabushiki Kaisha
   <120> NUCLEIC ACID PCR AMPLIFICATION METHOD, PCR PRIMER SET, PCR AMPLIFICATION PRODUCT, AND METHOD F0 R DETECTION OF NUCLEIC ACID USING THE AMPLIFICATION METHOD
<130> 2915330
<140> CF018137
   <141> 2004-05-19
<150> JP 2003/140836
   <151> 2003-05-19
<150> JP 2003/140838
   <151> 2003-05-19
<160> 5
<170> Patentln version 3.1
<210> 1
   <211> 18
   <212> DNA
   <213> Artificial
<220>
   <223> Synthesized DNA probe P1
<400> 1
   ccttaacgtg agttttcg                                                18
<210> 2
   <211> 18
   <212> DNA
   <213> Artificial
<220>
   <223> Synthesized DNA for forward primer F1
<400> 2
   ccttaacgtg agttttcg 18
<210> 3
   <211> 20
   <212> DNA
   <213> Artificial
<220>
   <223> Synthesized DNA for forwad primer F2
<400> 3
   cccttaacgt gagttttcgt 20
<210> 4
   <211> 24
   <212> DNA
   <213> Artificial
<220>
   <223> Synthesized DNA for forward primer F3
<400> 4
   atcccttaac gtgagttttc gttc 24
<210> 5
   <211> 20
   <212> DNA
   <213> Artificial
<213> Artificial
<220>
   <223> Synthesized DNA for reverse primer R1
<400> 5
   gcggtaatac ggttatccac 20